# EUROPEAN PATENT APPLICATION

(11) **EP 3 691 129 A1**
(43) Date of publication of application: **05.08.2020**
(21) Application number: 19181438.3
(22) Date of filing: 20.06.2019
(51) Int. Cl.: H03K 17/955, D03D 1/00, D02G 3/44

(54) **SENSING ELEMENT AND SENSING CONTROL SYSTEM THEREOF**

(30) Priority: 31.01.2019 TW 108103846
(71) Applicant: Intelligence Textile Technology Co., Ltd., 10595 Taipei City (TW)
(72) Inventor: CHEN, Shih-Hsiu, 10595 Taipei City (TW); FAN, Ching-Feng, 231 New Taipei City (TW)
(74) Representative: Perani & Partners S.p.A.

(57) **Abstract**

A sensing element (1, 2, 410) has a signaling yarn (110, 210, 300) and a single core wire (120, 220) that is intersecting with the signaling yarn (110, 210, 300). A sensing control system (4) includes the sensing element (1, 2, 410), a sensing control module (421), and a central control module (422). The sensing control module (421) is configured to output a scanning signal to the sensing element (1, 2, 410), receive a sensing signal corresponding to the sensing component, and generate a sensing result according to the sensing signal. The central control module (422) is electrically connected to the sensing control module (421), and is configured to control a component according to the sensing result. Thereby, the sensing element (1, 2, 410) of the embodiment of the application can be manufactured by a simple process and has a relatively small volume, and achieve the sensing operation in conjunction with the sensing control system (4).

## Description

### BACKGROUND OF THE INVENTION

### 1. Field of the Invention

The present invention relates to a sensing element, and more particularly, to a sensing element for sensing whether an object approaches or touches and a sensing control system thereof.

### 2. Description of the Related Art

Most smart textiles consist of a control circuit, yarns, wires (e.g., enameled wires) and resistors. The wires are embedded or woven in a textile woven from yarns and are connected to the resistors and control circuit. The wires can be connected to an external power source, and provide power of the external power source to the resistors and control circuit. When the resistors are powered, electric energy is converted into heat energy to heat a smart garment.

Smart textiles can further include a switch element for generating control signals to the control circuit. However, the switch element can be an electronic switch device having a housing and occupies a certain volume, and can only be manufactured through a complicated fabrication process.

### BRIEF SUMMARY OF THE INVENTION

In view of the above prior art, the present invention provides a sensing element and a sensing control system thereof, so as to achieve objects of a simple manufacturing process and a light, slim product occupying a minimal space.

To achieve the above and other objects, a sensing element is provided according to an embodiment of the present invention. The sensing element includes a signaling yarn and a single core wire. The signaling yarn includes a signaling yarn sensing portion which includes at least one bent unit and at least two signaling units connected to the at least one bent unit. The single core wire is configured at the signaling yarn sensing portion, and intersects with the at least two signaling units. Thereby, the sensing element according to the embodiment of the present invention can be manufactured by a simple process and has a relatively small volume.

In one embodiment, the at least two signaling units extend along a first direction and are arranged in a second direction.

In one embodiment, two of the signaling units are spaced by a distance of 1 mm.

In one embodiment, the single core wire extends along the second direction.

In one embodiment, the sensing signal is outputted by one of the signaling yarn and the single core wire.

In one embodiment, the signaling yarn includes a short spun fiber and a plate conductor. The strength of the short spun fiber is between 26 and 40 deniers. The plate conductor spirally encircles the surface around the short spun fiber. Accordingly, the sensing element according to the embodiment of the present invention has a certain level of strength and can thus be configured at a flexible material (e.g., a textile) and can mitigate the issue of disengagement caused by pulling or washing.

In one embodiment, the plate conductor is made of a material selected from one of the following: a copper-nickel alloy, a copper-tin alloy, a copper-nickel-silicon alloy, a copper-nickel-zinc alloy, a copper-nickel-tin alloy, a copper-chromium alloy, a copper-silver alloy, a nickel-brass alloy, a phosphor bronze alloy, a beryllium-copper alloy, a nickel-chromium alloy, a copper-tungsten alloy and a stainless steel.

To achieve the above and other objects, the present invention provides a sensing element including a single core wire and a plurality of signaling yarns. The single core wire has a single core wire sensing portion which includes at least one bent unit and at least two single core units connected to the at least one bent unit. The plurality of signaling yarns are configured at the single core wire sensing portion and intersect with the at least two single core units. Thereby, the sensing element according to the embodiment of the present invention can be manufactured by a simple process and has a relatively small volume.

In one embodiment, the single core wire is configured to output a sensing signal.

In one embodiment, the plurality of signaling yarns extends in a first direction and is arranged in a second direction.

In one embodiment, two of the signaling units are spaced by a distance of 1 mm.

In one embodiment, the at least two single core units are arranged along the first direction.

In one embodiment, the signaling yarn includes a short spun fiber and a plate conductor. The strength of the short spun fiber is between 26 and 40 deniers, and the short spun fiber acts as a supporting material. The plate conductor spirally encircles the surface around the short spun fiber. Accordingly, the sensing element according to the embodiment of the present invention has a certain level of strength and can thus be configured at a flexible material (e.g., a textile) and can mitigate the issue of disengagement caused by pulling or washing.

In one embodiment, the plate conductor is made of a material selected from one of the following: a copper-nickel alloy, a copper-tin alloy, a copper-nickel-silicon alloy, a copper-nickel-zinc alloy, a copper-nickel-tin alloy, a copper-chromium alloy, a copper-silver alloy, a nickel-brass alloy, a phosphor bronze alloy, a beryllium-copper alloy, a nickel-chromium alloy, a copper-tungsten alloy and a stainless steel.

To achieve the above and other objects, the present invention provides a sensing control system including the foregoing sensing element, a sensing control module and a central control module. The sensing control module is configured to receive a sensing signal and generate a sensing result according to the sensing signal. The central control module is electrically connected to the sensing control module, and is configured to control an element according to the sensing result. The sensing element according to the embodiment of the present invention can be manufactured by a simple process and has a relatively small volume, and can thus be flexibly configured to expand the application scope as well as improving utilization convenience of the sensing element and a sensing control system.

In one embodiment, the element is a Bluetooth® communication module.

In one embodiment, the element is a heating textile.

### BRIEF DESCRIPTION OF THE DRAWINGS

FIG. 1 is a schematic diagram of a sensing element according to an embodiment of the present invention.
FIG. 2 is a schematic diagram of a sensing element according to another embodiment of the present invention.
FIG. 3 is a perspective schematic diagram of a signaling yarn according to an embodiment of the present invention.
FIG. 4 is a cross-sectional schematic diagram of the signaling yarn according to an embodiment of the present invention.
FIG. 5 is a structural schematic diagram of a sensing control system according to an embodiment of the present invention.
FIG. 6 is a schematic diagram of the sensing control system configured at a smart textile according to an embodiment of the present invention.

### DETAILED DESCRIPTION OF THE PREFERRED EMBODIMENTS

To thoroughly understand the objects, features and effects of the present invention, specific embodiments are given with the accompanying drawings to describe the present invention in detail below.

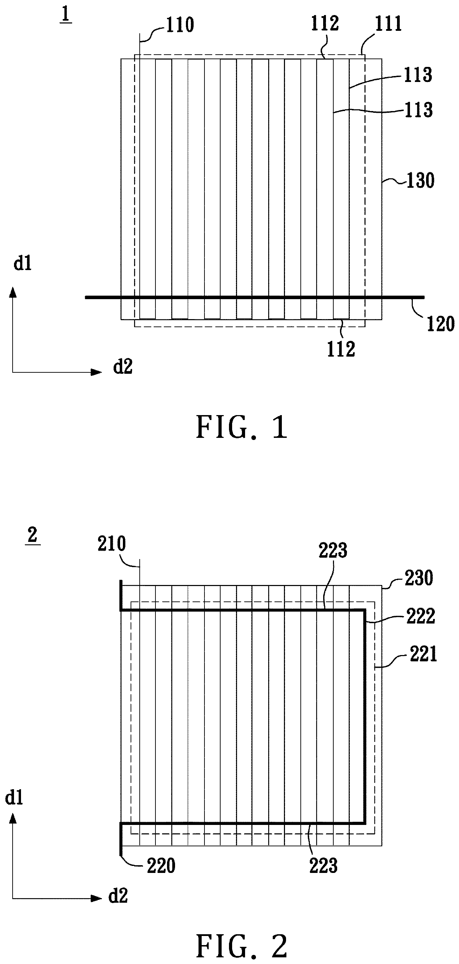

Referring to FIG. 1, FIG. 1 shows a schematic diagram of a sensing element 1 according to an embodiment of the present invention. The sensing element 1 is configured to generate a sensing signal, and includes a signaling yarn 110 and a single core wire 120.

The signaling yarn 110 includes a signaling yarn sensing portion 111 which includes at least one bent unit 112 and at least two signaling units 113 connected to the at least one bent unit 112. Each bent unit 112 is connected to two signaling units 113. The at least two signaling units 113 extend along a first direction d1 and are arranged in a second direction d2, wherein the first direction d1 is perpendicular to the second direction d2. In this embodiment, the signaling yarn 110 is a single signaling yarn, and thus the quantity of the signaling units 113 is determined according to the quantity of the bent units 112, and the quantity of the bent units 112 is determined according to requirements (e.g., the sensing sensitivity). In one embodiment, two signaling units 113 are spaced by a distance of, for example but not limited to, 1 mm; however, the distance between two signaling units 113 can be determined according to requirements (e.g., the sensing sensitivity).

The single core wire 120 is configured at the signaling yarn sensing portion 111, and intersects with the signaling units 113. In this embodiment, the single core wire 120 extends along the second direction d2 and is perpendicular to the signaling units 113.

In this embodiment, the signaling yarn 110 and the single core wire 120 are fixedly sewn to a textile 130, which is, for example, a textile woven from common yarns. In one embodiment, the textile 130 is, for example, quadrilateral, and has sides of 30 mm; however, the present invention is not limited to the above examples.

In this embodiment, the sensing signal can be outputted by one of the signaling yarn 110 and the single core wire 120.

Referring to FIG. 2, FIG. 2 shows a schematic diagram of a sensing element 2 according to another embodiment of the present invention. The sensing element 2 includes a plurality of signaling yarns 210 and a single core wire 220.

The single core wire 220 includes a single core wire sensing portion 221 which includes at least one bent unit 222 and at least two single core units 223 connected to the at least one bent unit 222. The bent unit 222 is connected to two single core units 223. The at least two single core units 223 are arranged in the first direction d1 and are parallel to the second direction d2, and the first direction d1 is perpendicular to the second direction d2. In this embodiment, the single core wire 220 is configured to output a sensing signal.

The plurality of signaling yarns 210 extends in the first direction d1, is arranged along the second direction d2 and configured at the single core wire sensing portion 221, and intersects with the at least two single core units 223. For example, each signaling yarn 210 can be substantially perpendicular to the at least two single core units 223. In one embodiment, a distance between two signaling yarns 210 is, for example but not limited to, 1 mm.

In this embodiment, the plurality of signaling yarns 210 and the single core wire 220 are fixedly sewn to a textile 230. For example, the textile 230 is a textile woven from common yarns. In one embodiment, the textile 230 is, for example, quadrilateral, and has sides of 30 mm; however, the present invention is not limited to the above examples.

A capacitor can be formed between the signaling yarn 110 (210) and the single core wire 120 (220) included in the sensing element 1 (2) according to the embodiments of the present invention. When an object (e.g., a finger) approaches or touches the sensing element (equivalent to a sensing event), the value of the capacitor is changed as a result of the change in an electric field due to the approaching or touching of the object. Thus, on the basis of a scanning signal received and a sensing signal correspondingly generated by the sensing element 1 (2), whether the value of the capacitor is changed can be determined according to the sensing signal to further determine whether a sensing event takes place (whether an object approaches or touches the sensing element).

In one embodiment, the single core wire 120 (220) can be implemented by a copper-tin alloy clad by an insulation layer, wherein the insulation layer can be made of an insulating varnish material selected from one of the following: polytetrafluoroethylene, polyethylene terephthalate, fluoroplastic film, polyvinyl chloride, polyethylene and other polymer insulation materials; however, the present invention is not limited to the above examples.

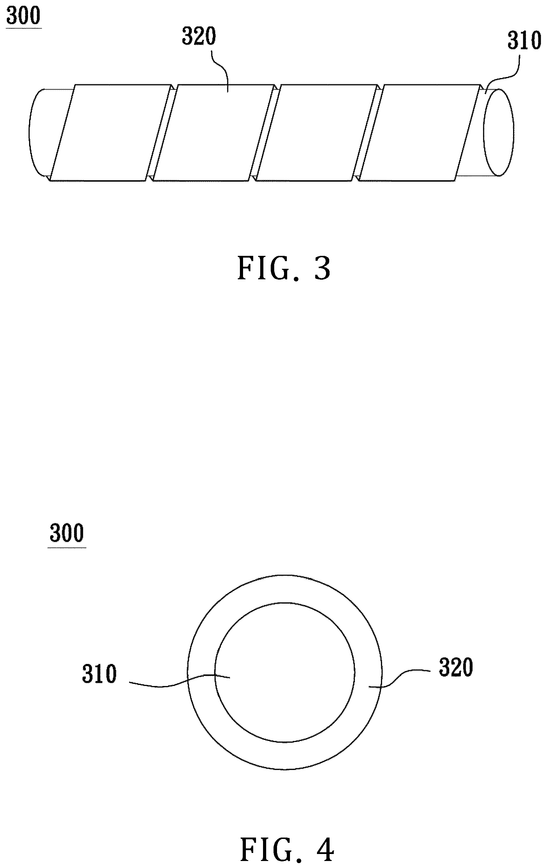

Referring to FIG. 3 and FIG. 4, FIG. 3 and FIG. 4 show schematic diagrams of a signaling yarn 300 according to embodiments of the present invention. The signaling yarn 300 includes a short spun fiber 310 and a plate conductor 320. The short spun fiber 310 acts as a supporting material to support the plate conductor 320 around the short spun fiber 310. The plate conductor 320 spirally encircles the surface around the short spun fiber 310. By spirally encircling the surface around the short spun fiber 310 by the plate conductor 320, the tensile strength of the signaling yarn 300 can be increased.

Selectively, the strength of the short spun fiber 310 can be selected and/or the length-width ratio of a cross section corresponding to a spiral direction of the plate conductor 320 can be selected to further increase the tensile strength of the signaling yarn 300. In this embodiment, the strength of the short spun fiber 310 is selectively 30 deniers, and the length-width ratio of the cross section corresponding to the spiral direction of the plate conductor 320 is selected to be approximately 20; however, the present invention is not limited to the above examples. For example, the strength of the short spun fiber 310 can be selectively 26, 28 or 40 deniers; alternatively, the length-width ratio of the cross section corresponding to the spiral direction of the plate conductor 320 can be selectively between 10 and 30.

In this embodiment, the short spun fiber 310 is made of a material selected from one of the following: polyesters, polyamides, polyacrylonitriles, polyethylenes, polypropylenes, celluloses, proteins, elastic fibers, polyperfluoroethylenes, poly-p-phenylene benzobisthiazoles, polyether ketones, carbons and glass fibers; however, the present invention is not limited the above examples. The material of the short spun fiber 310 can be selected according to actual requirements.

In this embodiment, the plate conductor 320 can be made of an alloy material, for example, a material selected from one of the following: a copper-nickel alloy, a copper-tin alloy, a copper-nickel-silicon alloy, a copper-nickel-zinc alloy, a copper-nickel-tin alloy, a copper-chromium alloy, a copper-silver alloy, a nickel-brass alloy, a phosphor bronze alloy, a beryllium-copper alloy, a nickel-chromium alloy, a copper-tungsten alloy, a stainless steel and other commercial conductive alloys; however, the present invention is not limited to the above types of alloys. In different applications, different types of alloys can be selected.

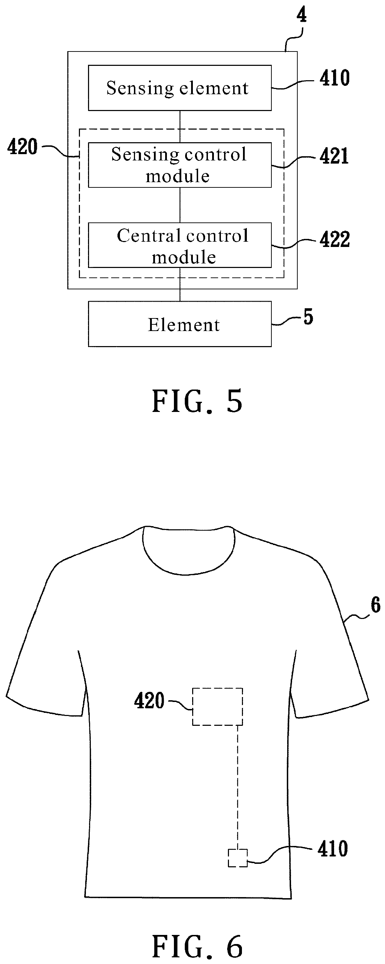

FIG. 5 shows a structural schematic diagram of a sensing control system according to an embodiment of the present invention. The sensing control system 4 includes a sensing element 410 and a control module 420. The control module 420 includes a sensing control module 421 and a central control module 422.

The sensing control module 421 is configured to generate a scanning signal provided to the sensing element 410, receive a sensing signal generated correspondingly to the scanning signal, and provide a sensing result according to the sensing signal. In one embodiment, the sensing control module 421 is, for example but not limited to, a capacitor sensing chip.

The central control module 422 is electrically connected to the sensing control module 421, and is configured to receive the sensing result and determine whether to control an electrically connected element 5 or perform a corresponding operation according to the sensing result. For example, the central sensing module 422 determines according to the sensing result whether a sensing event takes place, and accordingly controls the element 5 when the central control sensing module 422 determines that a sensing event takes place. In one embodiment, the central control module 422 is, for example but not limited to, a control circuit.

In one embodiment, the sensing element 410 can be configured at an electronic device such as a smartphone, and the central control module 422 can perform a corresponding operation according to the sensing result. For example, when the central control module 422 determines that a sensing event takes place, the central control module 422 controls the smartphone to display a screen of a locked state.

In one embodiment, the element 5 is, for example, a Bluetooth® communication module. Thus, in this embodiment, the central control module 422 can control the Bluetooth® communication module to perform a corresponding operation according to the sensing result. For example, when the central control module 422 determines that a sensing event takes place, the central control module 422 controls the Bluetooth® communication module to send a signal and to establish a communication connection with an electronic device, wherein the electronic device is a smartphone; however, the present invention is not limited to the above example.

In one embodiment, the element 5 is, for example, a heating textile, and includes at least one resistor element. Thus, in this embodiment, the central control module 422 can control the heating textile to heat up according to the sensing result. For example, when the central control module 422 determines that a sensing event takes place, the central control module 422 provides a current to the resistor element in the heating textile, and the temperature of the resistor element then rises by means of the current flowing through to heat up the heating textile.

In one embodiment, the sensing control system 4 can be configured at, for example but not limited to, a smart textile. As shown in FIG. 6, the sensing element 410 can be configured at a smart textile 6 near the arm or the palm, and be electrically connected to the control module 420 through a signaling yarn or other conductive wires, and a user can conveniently perform the operations described above (e.g., causing the smart textile to control the heating textile to heat up by the sensing of the sensing element 410) by approaching or touching the sensing element with a finger or a palm; however, the present invention is not limited to the above operation.

In one embodiment, only when the central control module 422 continuously determines that the sensing result indicates that a sensing event takes place and the sensing event continuously determined as taking place lasts for over 3 seconds, the central control module 422 then controls the element 5 or performs a corresponding operation, so as to prevent the sensing element 410 unintentionally touched from causing an event of the sensing control system 4 performing a corresponding operation. Thus, it is ensured that the sensing control system 4 correctly operates according to an operation of a user. In the above, the value "3 seconds" is merely an example, and the present invention is not limited to this example.

In conclusion, the sensing element according to embodiments of the present invention can be completed by only the signaling yarn and the single core wire, hence significantly reducing the manufacturing complexity and the overall volume of the sensing element. Furthermore, with the improved tensile strength provided by the signaling yarn, the sensing element according to embodiments of the present invention can be configured at a flexible material, further expanding the application scope and the configuration flexibility of the sensing element, thereby enhancing commercial values and utilization convenience of the present invention.

## Claims

1. A sensing element (1,410), configured to generate a sensing signal, comprising:
a signaling yarn (110, 300), comprising a signaling yarn sensing portion (111), the signaling yarn sensing portion (111) comprising at least one bent unit (112) and at least two signaling units (113) connected to the at least one bent unit (112); and
a single core wire (120), configured at the signaling yarn sensing portion (111), intersecting with the at least two signaling units (113).

2. The sensing element (1, 410) according to claim 1, wherein the at least two signaling units (113) extend along a first direction and are arranged in a second direction.

3. The sensing element (1, 410) according to claim 2, wherein two of the signaling units (113) are spaced by a distance of 1 mm.

4. The sensing element (1, 410) according to claim 2, wherein the single core wire (120) extends along the second direction.

5. The sensing element (1, 410) according to claim 1, wherein the sensing signal is outputted by one of the signaling yarn (110, 300) and the single core wire (120).

6. The sensing element (1, 410) according to claim 1, wherein the signaling yarn (110, 300) comprises:
a short spun fiber (310), having a strength between 26 and 40 deniers; and
a plate conductor (320), spirally encircling a surface around the short spun fiber (310).

7. The sensing element (1, 410) according to claim 6, wherein the plate conductor (320) is made of a material selected from one of the following: a copper-nickel alloy, a copper-tin alloy, a copper-nickel-silicon alloy, a copper-nickel-zinc alloy, a copper-nickel-tin alloy, a copper-chromium alloy, a copper-silver alloy, a nickel-brass alloy, a phosphor bronze alloy, a beryllium-copper alloy, a nickel-chromium alloy, a copper-tungsten alloy and a stainless steel.

8. A sensing element (2, 410), comprising:
a single core wire (220), comprising a single core wire sensing portion (221), the single core wire sensing portion (221) comprising at least one bent unit (222) and at least two single core units (223) connected to the at least one bent unit (222); and
a plurality of signaling yarns (210, 300), configured at the single core wire sensing portion (221), intersecting with the at least two single core units(223).

9. The sensing element (2, 410) according to claim 8, wherein the single core wire (220) is configured to output a sensing signal.

10. The sensing element (2, 410) according to claim 8, wherein the plurality of signaling yarns (210, 300) extends in a first direction and is arranged along a second direction.

11. The sensing element (2, 410) according to claim 10, wherein two of the signaling yarns (210, 300) are spaced by a distance of 1 mm.

12. The sensing element (2, 410) according to claim 10, wherein the at least two single core units (223) are arranged along the first direction.

13. The sensing element (2, 410) according to claim 8, wherein each of the signaling yarns (210, 300) comprises:
a short spun fiber (310), having a strength between 26 and 40 deniers; and
a plate conductor (320), spirally encircling a surface around the short spun fiber(310).

14. The sensing element (2, 410) according to claim 13, wherein the plate conductor (320) is made of a material selected from one of the following: a copper-nickel alloy, a copper-tin alloy, a copper-nickel-silicon alloy, a copper-nickel-zinc alloy, a copper-nickel-tin alloy, a copper-chromium alloy, a copper-silver alloy, a nickel-brass alloy, a phosphor bronze alloy, a beryllium-copper alloy, a nickel-chromium alloy, a copper-tungsten alloy and a stainless steel.

15. A sensing control system (4), comprising:
the sensing element (1, 2, 410) according to any one of claims 1 to 14;
a sensing control module (421), configured to receive the sensing signal, and generate a sensing result according to the sensing signal; and
a central control module (422), electrically connected to the sensing control module (421), configured to control an element (5) according to the sensing result.
